# EUROPEAN PATENT APPLICATION

(11) **EP 4 623 899 A1**
(43) Date of publication of application: **01.10.2025**
(21) Application number: 24382321.8
(22) Date of filing: 25.03.2024
(51) Int. Cl.: A61K 9/00, A61K 9/16, A61K 9/20, A61K 31/00

(54) **SOLID COMPOSITIONS COMPRISING CHLORHEXIDINE DIGLUCONATE AND/OR DIACETATE AND CETYLPYRIDINIUM CHLORIDE**

(71) Applicant: DENTAID, S.L., 08290 Cerdanyola del Vallès (ES)
(72) Inventor: MASSOLI, Alberto, Barcelona (ES); PUIG, Gemma, Barcelona (ES); LEÓN BERRÍOS, Rubén, Sant Cugat del Vallès (ES); BLANC POCIELLO, Vanessa, Barcelona (ES); GISPERT RIBAS, Juan, Zaragoza (ES)
(74) Representative: Ponti & Partners, S.L.P

(57) **Abstract**

The present invention relates to a solid composition comprising chlorhexidine digluconate and/or diacetate and cetylpyridinium chloride for use in the prevention and/or treatment of oral infectious pathologies.

## Description

### Field of the invention

The present invention relates to the field of oral pathologies. In particular, the present invention relates to a solid composition comprising chlorhexidine digluconate and/or diacetate and cetylpyridinium chloride for use in the prevention and/or treatment of oral infectious pathologies.

### Background of the invention

The use of Chlorhexidine (CHX) in the treatment of infectious diseases of the oral cavity or after operative procedures in this location is widely known. Similarly, the use of cetylpyridinium chloride (CPC) (or other quaternary ammonium salts) for the daily control of the microbial and viral load in the mouth to prevent possible infections is well known. There are numerous oral hygiene products on the market formulated with these active ingredients, mostly mouth rinses.

The efficacy of these products is directly proportional to the "availability" of the antiseptic contained therein for their action. The term "availability" of an antiseptic means its ability to reach its preferred target. In the case of the oral cavity, this target refers to the pathogenic micro-organisms that grow in saliva and in the form of biofilm on the different oral surfaces. In this respect, the different elements of the formulation have an important influence on the availability of the antiseptic.

Properly designed and formulated liquid formulations containing CHX or CHX and CPC are effective in the treatment of infectious diseases of the oral cavity, while the daily application of CPC products supports the maintenance of an optimal periodontal health.

Despite the obvious usefulness of using mouthwashes, according to the prescribed dosage, as a means of prevention/treatment of pathologies of the oral cavity, they have a number of disadvantages, which are listed below.

First of all, environmental concerns can be mentioned. In the composition of these types of formulations the major component is water at a usual % of 80 - 85 % depending on the type of product, while about 10 % is constituted by a humectant/solvent system based on the use of polyols (glycerine, sorbitol etc.) and short-chain glycols (propylene glycol, pentylene glycol etc.). Only the remaining about 5% includes sweeteners, colouring agents, preservatives, flavourings, emulsifiers and active ingredients.

The water used in the manufacture of mouthwashes is not only the water that goes directly into the composition of these products, but also includes the water needed in the washing and sanitising processes of all the machinery (reactors/mixers, pumps, filtration tools, intermediate storage tanks, packaging machinery and all the necessary piping network) used in the manufacture of these products.

The amount of water involved in all the manufacturing processes of a mouthwash is usually high and is estimated at 3.0 kg per 1.0 kg of manufactured product. This water must have certain well-defined characteristics in order to be used in the manufacture of these products, notably: absence of salts, chemical contaminants, chlorine, dissolved organic substances and high microbiological purity. The raw material for obtaining water with these characteristics is usually tap water for human consumption.

The treatment plant for this tap water usually includes high-tech and costly devices such as reverse osmosis, microfilters and sterilisation devices using ultraviolet rays usually generated by high-power UV lamps. Once water with the desired characteristics is obtained, it must be stored in appropriate tanks at a temperature below 20 °C and kept in constant turbulent recirculation by a pump system and continuously irradiated with high-power UV rays. If it is not used within 72 hours, it must be disposed of by controlled discharge into the sewage system.

The entire production/recirculation system of the water used in the manufacture of mouthwashes is constantly monitored to detect possible microbiological contamination or other types of incidents. All the manufactured water circuits are cleaned and maintained on a regular basis.

For all the above reasons, the process of production, maintenance and control of the water used in the manufacture of mouthwashes is costly, complicated and has a clear impact on the environment.

Another aspect to be considered in current mouthwash manufacturing technology, due to the high percentage of water they contain, is the need to add a suitable preservative system to the formula to prevent/remove possible microbiological contamination during use. In this regard, we can mention the case of parabens, which are a group of chemical compounds commonly found in personal care products. Although there is much discussion thereabout, this type of product has been considered by some authors as a potential carcinogen.

The palletised obtained product is usually bulky and heavy, generating a significant logistical effort with the consequent high energy consumption (fossil fuels and electrical energy). The packaging of mouthwashes requires the use of packaging material of a consistent volume (mass) consisting mainly of plastic material that is not always entirely recycled or recyclable.

In short, the environmental problem generated by the manufacture, distribution and use of water-based mouthwashes can be summarised in the following points:
- High need for treated water for the manufacture of mouthwashes (3.0 kg of water consumed per 1.0 kg of product manufactured) and, therefore, subtraction of large volumes of tap water suitable for human consumption from the population when it is already a very limited resource.
- High amount of energy used in obtaining water suitable for the manufacture of mouthwashes and in its conservation process until its use.
- Use of chemical agents during the process of conditioning the water for the manufacture and in the maintenance of the production facilities thereof.
- High amount of energy used in the manufacture and conditioning of large volumes of mouthwash.
- The need to add preservatives to mouthwashes whose long-term effects on micro-organisms in the environment are not yet fully evaluated.
- Transport and distribution of bulky and heavy mouthwashes, which is a major logistical effort, costly and fossil fuel-intensive.
- Need for packaging including bottles (150 - 500 ml), caps, dispensing cups; this material is not always fully recyclable or recycled.

On the other hand, the aetiological agents causing disease in the oral cavity are, in most of cases, bacterial in nature. These pathogenic bacteria, in oral health situations, are usually present in a low load, resulting in the oral biofilm being mainly dominated by commensal bacteria that are in homeostasis with the host. Whether oral bacteria grow in biofilm adhering to surfaces or localised in saliva, they are always aggregated.

Oral biofilm forms and adheres to tooth surfaces according to a well-defined sequence of events and is responsible for numerous diseases, from dental caries to periodontal diseases (gingivitis and periodontitis) and peri-implant diseases (mucositis and peri-implantitis).

Within this oral biofilm, pathogenic bacteria are not very accessible to the action of antiseptics, as they are usually located in the deepest layers of the biofilm and are additionally protected by an extracellular matrix composed of polysaccharides, proteins and DNA. As a result of the metabolism of the different bacterial species that make up the supragingival and subgingival biofilm, pathologies such as dental caries are induced, triggered by the secretion of organic acids, or the degradation of the gingival epithelium, caused by the release of proteases and toxins and the consequent inflammatory response.

Oral biofilm evolves and its biological vitality may change. Once it is "mature" it can be mineralised and form dental calculus. Its accumulation at the gingival margin can induce inflammation as well as aesthetic problems. It can only be removed by mechanical action.

It follows that the key to oral biofilm control lies in the possibility of reaching and destroying the pathogenic species present in this structure by means that are easily applicable and available to all.

Despite this need, most of mouthwashes on the market contain antiseptic molecules that are unable to degrade the extracellular matrix and disintegrate the pathogenic oral biofilm. At best, the antiseptic limits its action to the most superficial layers of the oral biofilm as evidenced by confocal light microscopy studies. Many formulations are unable to penetrate beyond one third of the thickness of the biofilm.

The consequence of this is an insufficient control of oral biofilm and the tendency of mouthwash manufacturers to increase the concentration of antiseptic, which in turn increases the side effects of their use in the oral cavity (staining, loss of taste, etc.).

In order to solve all the above problems, the present inventors have developed a solid composition comprising chlorhexidine digluconate and/or diacetate and cetylpyridinium chloride. Such a solid composition can be converted into mouthwashes or liquids for application by dental irrigators by simple extemporaneous dissolution in suitable volumes of tap or bottled water or even such a composition can also be administered directly into the oral cavity.

In addition, the manufacturing technology of these solid formulations involves the use of a very small amount of deionised water (substantially washing and sanitising processes of the machinery), obtained by treatment of the tap water, which can be estimated at a maximum of 10% by weight of the produced solid formulation and not 300% as in the case of a classic mouthwash. This significantly reduces the consumption of tap water, energy consumption and the use of chemical agents related to the water manufacturing process and the sanitation of the reactors.

As indicated above, the presence of preservatives is necessary in water-based mouthwashes due to the large amount of water present, which facilitates the development of contaminating microbial species (bacteria, fungi or yeasts) that endanger the integrity of the product and the patient's health. On the other hand, the solid formulations of the present invention, although manufactured to avoid accidental microbiological contamination, allow the use of preservatives to be avoided, taking into account two essential facts:
- The amount of water present in these preparations is very low. Thus, the proliferation of possible microbiological contamination is very unlikely as the conditions for bacterial growth are not present.
- These compositions will typically be supplied as single doses and are consumed in an extremely short time once dissolved in tap or bottled water.

Another significant advantage is the significant reduction of the environmental impact attributable to these solid formulations in terms of transport and distribution, as the handled volumes and weights are much lower, being reduced by 90% compared to a regular mouthwash. The packaging can also be made using more environmentally friendly materials and will be individual for each dose.

### Summary description of the invention

The present invention relates to a solid composition comprising chlorhexidine digluconate and/or chlorhexidine diacetate and cetylpyridinium chloride for use in the prevention and/or treatment of oral infectious pathologies.

### Brief description of the figures

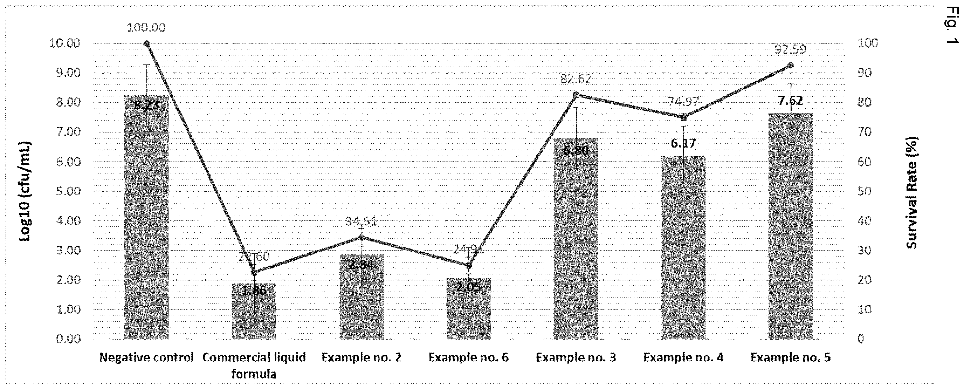

Figure 1 shows the total bacterial counts in cfu/mL and the survival rate of biofilms after treatment with various solid compositions with CHX and CPC and the *gold* standard liquid formulation (reference liquid formulation which is a commercial mouthwash with CHX at 12 % w/w and CPC at 0.05 % w/w).

### Detailed description of the invention

The present invention relates to a solid composition comprising chlorhexidine digluconate and/or diacetate and cetylpyridinium chloride, for use in the prevention and/or treatment of oral infectious pathologies. In one embodiment, the present invention relates to a solid composition comprising chlorhexidine digluconate and cetylpyridinium chloride for use in the prevention and/or treatment of oral infectious pathologies. In another embodiment, the present invention relates to a solid composition comprising chlorhexidine diacetate and cetylpyridinium chloride for use in the prevention and/or treatment of oral infectious pathologies. In another embodiment, the present invention relates to a solid composition comprising chlorhexidine digluconate, chlorhexidine diacetate and cetylpyridinium chloride for use in the prevention and/or treatment of oral infectious pathologies.

In this disclosure and in the claims, terms such as "comprises", "comprising", "containing" and "having" are open terms and may mean "includes", "including" and the like; whereas terms such as "consisting of" or "consists of" refer to the elements mentioned after these terms and exclude others which are not mentioned.

Unless otherwise explained, all technical and scientific terms used in this document have the same meaning as commonly understood by a person skilled in the art to which this disclosure pertains. The singular terms "a", "an" and "the" include plural referents unless the context clearly indicates otherwise. For example, if "a flavouring" is indicated, it is understood that it may be one or more flavourings. Similarly, the word "or" is intended to include "and" unless the context clearly indicates otherwise.

It should be noted that the term "about" as applied to the values used previously and subsequently in this document includes a margin of error of ± 5 %, such as ± 4 %, ± 3 %, ± 2 %, ± 1 %.

As used herein, the term "treat" or "treatment" or "treating" and the like refer to an improvement of at least one discernible symptom of the oral infectious pathologies listed herein. In another embodiment, "treat" or "treatment" or "treating" and the like refer to an improvement of at least one measurable physical parameter, not necessarily discernible by the patient. In another embodiment, "treat" or "treatment" or "treating" and the like refer to inhibiting the progression of at least one discernible symptom of the oral infectious pathologies listed herein. In another embodiment, "treat" or "treatment" or "treating" and the like refer to slowing the progression or reversing the progression of at least one discernible symptom of the oral infectious pathologies listed herein. As used herein, "prevent" or "prevention" or "preventing" and its cognates refer to delaying the onset or reducing the risk of acquiring or recurrence of at least one discernible symptom of the oral infectious pathologies listed herein. The general terms "prevent" or "prevention" or "preventing" and the like also include the prevention of recurrence in subjects who have had previous episodes of oral infectious pathologies listed herein.

As used herein, the term "prevention and/or treatment" includes "prevention and treatment" and "prevention or treatment".

In a preferred embodiment, said solid composition is in the form of a powder, granulate or tablet.

In another preferred embodiment, when the solid composition of the present invention comprises chlorhexidine digluconate as the only chlorhexidine salt and the CPC, the chlorhexidine digluconate is present at a concentration of 0,220-2,200 % w/w with respect to the total solid composition.

In another preferred embodiment, when the solid composition of the present invention comprises chlorhexidine diacetate as the only chlorhexidine salt and the CPC, chlorhexidine diacetate is present at a concentration of 0.154-1.540 % w/w with respect to the total solid composition.

In another preferred embodiment, when a mixture of chlorhexidine diacetate and chlorhexidine digluconate are used in the solid composition of the present invention as chlorhexidine salts in addition to CPC, the sum of the amount of each type of salt shall in no case exceed the concentration of CHX expressed as CHX digluconate of 0,220 - 2,200 % w/w with respect to the total solid composition.

In another preferred embodiment, cetylpyridinium chloride is present at a concentration of 0,133-1,100 % w/w with respect to the total solid composition.

For the constitution of the solid composition of the present invention, common components in the art for the formulation of solid compositions can be added, such as one or more solid compressible polyols, one or more solid or semi-solid emulsifiers and one or more calcium ion complexing agents. More optionally, the solid composition of the present invention further comprises a flavouring, a sweetener, a fluidising agent and mixture thereof. Preferably, and independently, the solid or semi-solid emulsifier is present at a concentration of 0.5-5.0 % w/w, the flavouring is present at a concentration of 0.55-2.20 % w/w, the sweetener is present at a concentration of up to 0.110 % w/w, the colouring agent is present at a concentration of up to 0.165 % w/w, the calcium ion complexing agent is present at a concentration of 1.50-30.00 % w/w and the fluidising agent is present at a concentration of up to 10 % w/w. The solid compressible polyol will be in the q.s. 100 % w/w.

In another preferred embodiment, the solid composition of the present invention is dissolved in water for reconstitution into a liquid or mouthwash for extemporaneous use or, alternatively, liquids for application by dental irrigators are prepared by simple extemporaneous dissolution in suitable volumes of tap or bottled water. Alternatively, in another preferred embodiment, the solid composition of the present invention is administered directly into the oral cavity. By way of example only, for the preferred concentrations of antiseptics indicated, the amount of solid to obtain adequate concentrations of CHX and CPC would be 1.5 grams to be employed as follows:

### Powder and granules:

a. 1.5 grams dissolved in 15 ml of tap water or bottled water without pre-treatment: the liquid obtained is used immediately and is analogous to a dose of the corresponding "standard" mouthwash with the same modalities for use.
b. 1.5 grams introduced directly into the oral cavity: allow to dissolve in the mouth and then the patient introduces water in the mouth and rinses as if it were a classic mouthwash expelling the remains.

30 grams dissolved in 300 ml of tap water or bottled water without pre-treatment: the liquid obtained is used immediately with a dental irrigator of suitable capacity. The amount of solid to be used will always be numerically equal to 10 % of the volume of water loaded in the irrigator device.

### Tablet:

a. One tablet is dissolved in 15 ml of tap water or bottled water without pre-treatment: the liquid obtained is used immediately and is analogous to a dose of the corresponding "standard" mouthwash with the same modalities for use.
b. A tablet is introduced directly into the oral cavity: it is fragmented and allowed to dissolve in the mouth. The patient then introduces water in the mouth and rinses as if it were a classic mouthwash expelling the remains.

In another preferred embodiment, oral infectious pathologies include, but are not limited to, caries in primary and/or permanent teeth, post-operative infections in the oral cavity, oral halitosis, gingivitis, periodontitis, oral mucositis, peri-implant mucositis, peri-implantitis, aphtha, fungi, sexually transmitted infections in the oral cavity, oral infectious pathologies affecting aspiration pneumonias in intubated and/or ICU (Intensive Care Unit) patients, oral infectious pathologies affecting aspiration pneumonias in patients suffering from Gastroesophageal Reflux, infected oral ulcers, herpetic gingivostomatitis.

The present disclosure also relates to a method for preventing and/or treating oral infectious pathologies according to the embodiments included herein, comprising the step of administering a therapeutically effective amount or dose of solid composition comprising chlorhexidine digluconate and/or chlorhexidine diacetate and cetylpyridinium chloride according to the embodiments included herein. The terms "therapeutically effective dose" and "therapeutically effective amount" are used to refer to an amount of the subject composition which results in the prevention, delay of the onset of symptoms or improvement of symptoms in the context of the present invention. A therapeutically effective amount as well as a therapeutically effective frequency of administration can be determined by methods known in the art.

It should be noted that any of the embodiments described herein may be considered alone or in combination with any other embodiment described herein, unless the context specifies otherwise. In other words, for example, a preferred option of one defined feature may be combined with a more or less preferred option of another feature.

The present invention will be now illustrated by means of an example which shall in no way be considered as limiting the invention.

### EXAMPLES

### Materials for the manufacture of the compositions of the invention indicated in A1-A3

- Stainless steel sieves suitable for homogenizing particle size of raw materials before mixing, during the process and in the final screening of the granulate.
- A mixer/pulveriser to ensure efficient mixing of the solids and homogenization of the size of the particles constituting the mixture.
- A kneading system for the incorporation of the CHX compound solution
- A volumetric dosing system suitable in size and accuracy for the incorporation of the calculated amount of the CHX solution of known density (g/ml) and concentration (w/w).
- An oven set at a temperature of 40 °C and with a vacuum for the drying phase of the product.
- A suitable packaging material for storing the granulate obtained prior to packaging.

### A1. Process for obtaining a composition comprising CHX digluconate and CPC

1. The selected, previously sieved, **compressible polyol (major component)** is loaded into the mixer/pulveriser.
2. The selected, previously sieved calcium ion **complexing agent** is loaded into the mixer/pulveriser. It is mixed at room temperature for 5 - 20 minutes.
3. The selected, previously sieved **solid emulsifier** is loaded into the mixer/pulveriser. It is mixed at room temperature for 5 - 20 minutes.
4. Optionally, the selected, previously sieved **CHX- and CPC-compatible sweetener** is loaded into the mixer/pulveriser. It is mixed at room temperature for 5 - 20 minutes.
5. The previously sieved **active ingredient CPC (Cetylpyridinium Chloride)** is loaded into the mixer/pulveriser. It is mixed at room temperature for 5 - 45 minutes.
6. Optionally, one or more previously sieved **additional components** are loaded into the mixer/pulveriser. It is mixed at room temperature for 5 - 45 minutes.
7. The solid mixture as obtained is extracted from the mixer/pulveriser and sieved.
8. The sieved solid is introduced into the kneading system and the expected volume of **CHX digluconate** solution is loaded into 4 separate fractions and mixed for 5 - 15 minutes after each addition.
9. The wet solid is dried at 40 °C under vacuum for 3 to 16 hours until complete removal of the added water from the CHX digluconate solution.
10. The dry solid as obtained is crushed and sieved. Optionally, the crushed and sieved solid from step 10 is put into the mixer/pulveriser. The expected amount of flavouring is added and mixed at room temperature for 5 - 20 minutes.
11. The solid mixture as obtained is extracted from the mixer/pulveriser and sieved.
12. The granulated powder as obtained is stored in a suitable packaging material.
13. Optionally, the granulate as obtained is directly compressed to obtain a tablet of a suitable size.

### A2. Procedure for obtaining a composition comprising CHX diacetate and CPC

1. The selected, previously sieved, **compressible polyol (major component)** is loaded into the mixer/pulveriser.
2. The selected, previously sieved calcium ion **complexing agent** is loaded into the mixer/pulveriser. It is mixed at room temperature for 5 - 20 minutes.
3. The selected, previously sieved **solid emulsifier** is loaded into the mixer/pulveriser. It is mixed at room temperature for 5 - 20 minutes.
4. Optionally, the intended amount of **flavouring** is loaded into the mixer/pulveriser and mixed at room temperature for 5 - 20 minutes.
5. Optionally, the selected, previously sieved **CHX- and CPC-compatible sweetener** is loaded into the mixer/pulveriser. It is mixed at room temperature for 5 - 20 minutes.
6. The previously sieved **active ingredient CPC (Cetylpyridinium Chloride)** is loaded into the mixer/pulveriser. It is mixed at room temperature for 5 - 45 minutes.
7. The previously sieved **active ingredient CHX (Chlorhexidine diacetate)** is loaded into the mixer/pulveriser. It is mixed at room temperature for 5 - 45 minutes.
8. Optionally, one or more previously sieved **additional components** are loaded into the mixer/pulveriser. It is mixed at room temperature for 5 - 45 minutes.
9. The solid mixture as obtained is extracted from the mixer/pulveriser and **sieved.**
10. The sieved solid from step 9 is fed back into the mixer/pulveriser. The internal temperature of the machine is adjusted to a value between 50 and 70 °C and it is mixed for a 5 - 45 minutes.
11. The temperature of the machine is regulated to 25°C. When this temperature is reached, it is mixed for between 5 and 30 minutes.
12. The solid mixture as obtained is extracted from the mixer/pulveriser and sieved.
13. **The granulated** powder as obtained is stored in a suitable packaging material.
14. Optionally, the granulate as obtained is directly compressed to obtain a tablet of a suitable size.

### A3: Procedure for obtaining a composition comprising a MIXTURE of CHX diacetate and CHX digluconate, and CPC

1. The selected, previously sieved, compressible polyol (major component) is loaded into the mixer/pulveriser.
2. The selected, previously sieved calcium ion complexing agent is loaded into the mixer/pulveriser. It is mixed at room temperature for 5 - 20 minutes.
3. The selected, previously sieved solid emulsifier is loaded into the mixer/pulveriser. It is mixed at room temperature for 5 - 20 minutes.
4. Optionally, the selected, previously sieved CHX- and CPC-compatible sweetener is loaded into the mixer/pulveriser. It is mixed at room temperature for 5 - 20 minutes.
5. The previously sieved active ingredient CPC (Cetylpyridinium Chloride) is loaded into the mixer/pulveriser. It is mixed at room temperature for 5 - 20 minutes.
6. The previously sieved active ingredient CHX (Chlorhexidine diacetate) is loaded into the mixer/pulveriser. It is mixed at room temperature for 5 - 45 minutes.
7. Optionally, one or more previously sieved additional components are loaded into the mixer/pulveriser. It is mixed at room temperature for 5 - 45 minutes.
8. The solid mixture as obtained is extracted from the mixer/pulveriser and sieved.
9. The sieved solid is introduced into the kneading system and the expected volume of CHX digluconate solution is loaded into 4 separate fractions and mixed for 5 - 15 minutes after each addition.
10. The wet solid is dried at 40 °C under vacuum for 3 to 16 hours until complete removal of the added water from the CHX digluconate solution.
11. The dry solid as obtained is crushed and sieved.
12. The crushed and sieved solid from step 11 is put into the mixer/pulveriser. The expected amount of flavouring is optionally added and mixed at room temperature for 5 - 20 minutes.
13. The solid mixture as obtained is extracted from the mixer/pulveriser and sieved.
14. The granulated powder as obtained is stored in a suitable packaging material.
15. Optionally, the granulate as obtained is directly compressed to obtain a tablet of a suitable size.

### B. Analysis of the products as obtained according to points A1 to A3

### Analysis of physical parameters

**1. Visual control of the homogeneity of the solid/granulates. APPROVED: the solid must be HOMOGENEOUS.**
**2. Solubility of the product in different types of water** (1.5 g of solid dissolved in 15 g of water). In particular, the solubility of the products is tested in tap water, bottled/treated mineral water WITHOUT GAS, deionised water of analytical grade. **APPROVED: a clear or slightly turbid solution must be obtained in all types of water used without suspended solids and/or residual precipitate.**
**3. Density (g/ml) of the solid:** this parameter is measured by weighing exactly 50 ml of the solid into a volumetric flask of the same volume. The density is indicated by the following ratio:
   d(g/ml) = Solid weight (g)/50 (volume of volumetric flask in ml). **APPROVED: value obtained between 0.500 and 0.800 g/ml.**
**4. pH** of the solution obtained by dissolving 1,5 g of solid in 15 g of deionised water of analytical grade. It is measured using an electronic pH meter with the corresponding calibrated electrode. **APPROVED: value obtained between 5,5 and 7.**

### Analysis of antiseptics (CHX and CPC)

The analysis of CHX and CPC is carried out using high resolution and sensitivity automated chromatographic systems, in particular HPLC (High Performance Liquid Chromatography) and/or UPLC (Ultra Performance Liquid Chromatography) systems. The titration of these active ingredients shall be carried out on the solution obtained by dissolving 1,5 g of solid in 15 g of deionised water of HPLC/UPLC quality. The obtained result shall be extrapolated to the solid.
1. CHLORHEXIDINE DI-GLUCONATE (CHX-dg):
   a. in solution: ± 10 % of the expected theoretical value (0.0200 - 0.200 % w/w)
   b. in the solid: ± 15 % of the expected theoretical value (0.2200 - 2.200 % w/w)
2. CHLORHEXIDINE DI-ACETATE (CHX-da):
   a. in solution: ± 10 % of expected theoretical value (0.0140 - 0.140 % w/w)
   b. in the solid: ± 15 % of the expected theoretical value (0.1540 - 1.540 % w/w)
3. CETYLPYRIDINIUM CHLORIDE
   a. in solution: ± 10 % of the expected theoretical value (0.0125 - 0.100 % w/w)
   b. in the solid: ± 15 % of the expected theoretical value (0.1375 - 1.100 % w/w)

### Analysis of the main impurity from CHX

The formation of p-chloroaniline (PCA) by CHX (CHX-da or CHX-dg) when this active ingredient is subjected to thermal stress (temperatures > 40°C) or unsuitable pH values (< 5.0) is well known in the art. The toxicity of this impurity is also well known. For this reason, only a maximum limit of 3 ppm is allowed in mouthwashes containing CHX.

The measurement of this impurity is carried out by using high resolution and sensitivity automated chromatographic systems and in particular HPLC and/or UPLC systems. The titration of these active ingredients shall be carried out on the solution obtained by dissolving 1,5 g of solid in 15 g of deionised water of HPLC/UPLC quality.

As stated above, the maximum amount of p-chloroaniline (PCA) present in the solution obtained by dissolving 1,5 g of solid in 15 g of deionised water of HPLC/UPLC quality sho be 3,0 ppm.

### Stability studies

In order to verify that the physical parameters, the concentrations of the antiseptics and the level of the PCA impurity (p-Chloroaniline) remain within the defined limits, a stability study is carried out at a temperature of 40°C (75 % RH) and then extrapolated to a temperature of 25°C.

The foreseen analytical procedures and limits are those already described for the analysis of the product described above. The conditions of 40°C and 75 % RH have been obtained using a climatic chamber calibrated and continuously controlled in temperature and humidity. To calculate the stability time at 40°C equivalent to 3 years at 25°C, the value of the kinetic parameter AAF (Accelerated Aging Factor) is used. For a stability temperature of 40°C and an extrapolation temperature T of 25°C the AAF value is 2.828. The time at 40 °C equivalent to 3 years (365*3 days) at 25 °C is therefore: (365*3)/2.828 = 387 days equivalent to 12.9 months.

In practice, 15 grams of the product to be analysed are placed in each of 13 borosilicate glass bottles of 100 ml of capacity, each fitted with an airtight lid of inert material and positioned vertically. The periodicity of analysis of the different bottles is monthly and defined in an appropriate calendar.

The obtained results, in terms of % recovery of the antiseptics and ppm of PCA, are plotted linearly versus time (days) assuming zero-order kinetics. With the data obtained, an equation of a straight line is obtained from which the time (years) in which the recovery of the antiseptics is 90 % at 40°C is calculated. To extrapolate this time to 25 °C, this time is multiplied by the AAF value, which is 2.828 in this case.

In the case of the PCA impurity, the time at 25°C necessary for the formation of the 3 ppm max. allowed in the solution obtained by dissolving 1,5 g of solid product in 15 g of water is calculated by a similar procedure.

This extrapolates the shelf life at 25°C of the solid products contained in the CHX - CPC combination.

### Measurement of antimicrobial activity

The overgrowth of periodontopathogenic bacteria in the gingival sulcus is the aetiological factor of periodontal diseases. Following the accumulation and active metabolism of pathogenic biofilm, clinical signs of inflammation, bleeding of gums, presence of periodontal pocket and bone degradation can confirm the development of periodontitis. Thus, control of bacterial loads in the different oral niches seems to be the key to prevent the establishment of such biofilm in the subgingival space. Daily mouthrinses should be formulated with antiseptic molecules capable of controlling the bacterial loads of oral pathogens. To meet this objective, Chlorhexidine (CHX) and Cetylpyridinium Chloride (CPC) have been mainly used in Europe and the USA.

The *in vitro* effectiveness of these mouthwashes has commonly been evaluated using planktonic bacterial cultures; however, the growth of microorganisms in the oral environment is mainly in the form of biofilm. Hence, the evaluation of the actual effectiveness of the product must be performed using a study model that simulates the microbial lifestyle that exists in the oral environment. For this purpose, a model of oral biofilm composed of commensal and periodontopathogenic bacteria has been used, which simulates a pathogenic subgingival biofilm in terms of its growth conditions.

Furthermore, the antibiofilm capacity of the compositions of the present invention is compared with the effect caused by a commercial liquid formulation, a standard *gold* mouthwash with CHX and CPC.

### Formation and growth of a biofilm for the evaluation of the antimicrobial capacity of solid compositions containing CHX and CPC as described herein

### PHASE 1: Biofilm formation and growth.

Monospecies liquid cultures of the oral bacterial species *Streptoccus oralis* (ATCC35037), *Actinomyces naeslundii* (ATCC19039), *Veillonella párvula* (ATCC 10790), *Fusobacterium nucleatum* (ATCC10953), *Porphyromonas gingivalis* (ATCC33277) and *Aggregatibacter actinomycetemcomitans* (ATCC33384) are grown in BHI (Brain Heart Infusion) liquid culture medium supplemented with haemin, menadione and glutamic acid (referred to as BHI-II) at 36.5 °C. Each species should be used when its growth is at the end of its exponential phase. Subsequently, a mixture of the cultures of the six species is made at a final concentration for each species of 10E3 cfu/mL for *S*. *oralis,* 10E4 cfu/mL for *A. naeslundi* and *V. parvula,* 10E5 cfu/mL for *F. nucleatum,* 10E6 cfu/mL for A. *actinomycetemcomitans* and *P*. *gingivales.* One mL of the mixture is inoculated into the wells of a microtiter plate containing sterile hydroxyapatite discs simulating the tooth surface. The inocula are incubated for 96 hours under anaerobiosis at 36,5 °C. After 48 hours, the culture medium is replaced; 700 µL are removed and 700 µL of BHI-II medium is added.

### PHASE 2: Treatment of the biofilms as obtained with the compositions of the present invention for the evaluation of their antiseptic activity.

After the incubation time, the biofilms grown on the hydroxyapatite discs are treated with a freshly prepared solution obtained by dissolving 1.5 g of the solid composition to be analysed in 15 g of water. In order to remove unattached cells, the biofilms are washed by immersing them in sterile phosphate buffered saline and then immersed in the dissolved composition for 2 minutes in mouthwash. Subsequently, the excess composition is removed by immersing the biofilms again in PBS. The treated biofilms are disaggregated by shaking for 5 minutes. The resulting bacterial suspension is serially diluted 1/10 in PBS and 0.1 mL of this suspension is seeded on Blood Agar No. 2 plates supplemented with haemin-menadione. These are incubated under anaerobiosis for 5 days. For recount of *A. actinomycentemcomitans* species, 0.1 mL of the serial dilutions are plated on solid medium containing bacitracin selective for this species and incubated in microaerophilia. Each formulation to be evaluated is tested on 25 biofilms grown on hydroxyapatite discs. The counts of the six species are presented in log 10 cfu/mL. Statistical analysis of the results is performed with the Mann-Whitney U test for significance comparative using the Jamovi software application, with the significance level set at 0.05.

### Examples of compositions. Compositions of the present invention and comparatives

### COMPARATIVE EXAMPLE 01:

Internal reference: **S/EML-03-00-A/01** (Product containing **CHX-dichlorohydrochloride** + **CPC)**

### Composition:

| | | |
|---|---|---|
| 1. | Xylitol | q.s. 100 % Xylitol |
| 2. | Sodium gluconate | 11.0 |
| 3. | Poloxamer 407 | 1.98 |
| 4. | Flavouring | 1.10% |
| 5. | Saccharin sodium | 0.11 %. |
| 6. | Chlorhexidine di-hydrochloride | 0.85 % Theoretical in reconstituted sol: 0.077 %. |
| 7. | CPC | 0.55 % Theoretical in reconstituted sol: 0.050 %. |

### ANALYTICAL RESULTS

Appearance: homogeneous white powder **(APPROVED)**
pH: **(NOT MEASURED BY PRESENCE OF UNDISSOLVED SOLID)**
Density of solid (g/ml): 0,650 **(APPROVED)**
Solubility (*): presence of undissolved crystalline solid **(NOT APPROVED)**
CHX-di-chlorohydrochloride (in reconstituted solution): **(NOT MEASURED)**

This composition presented a solubility problem due to the use of Chlorhexidine hydrochloride, which is not very soluble in aqueous media.

### EXAMPLE 02:

Internal reference: **S/EML-07-00-A/01** (Product with **CHX-dg** + **CPC)**

### Composition:

| | | |
|---|---|---|
| 1. | Xylitol | q.s. 100 |
| 2. | Sodium gluconate | 11.0 % |
| 3. | Poloxamer 407 | 1.98 % |
| 4. | Flavouring | 1.10% |
| 5. | Chlorhexidine di-gluconate | 1.32 % Theoretical in reconstituted sol.: 0.120 % |
| 6. | CPC | 0.55 % Theoretical in reconstituted sol.: 0.050 % |

### ANALYTICAL RESULTS

Appearance: homogeneous white powder **(APPROVED)**
pH: 6.61 (*) **(APPROVED)**
Density of solid (g/ml): 0.686 **(APPROVED)**
Solubility: **TRANSPARENT** solution in any kind of water **(APPROVED)**
CHX-dg (in reconstituted solution): 0.117 % (Recovery %: 97,5) **(APPROVED)**
CHX-dg (in the solid): 1.266 % (Recovery %: 95.9) **(APPROVED)**
CPC (in reconstituted solution): 0.047 % (Recovery %: 94.0) **(APPROVED)**
CPC (in the solid): 0.511 % (Recovery %: 92.9) **(APPROVED)**

### COMPARATIVE EXAMPLE 03:

Internal reference: **S/EML-10-00-A/01** (Product with **CHX-dg ONLY)** Composition:

| | | |
|---|---|---|
| 1. | Xylitol | q.s. 100 |
| 2. | Sodium gluconate | 11.0 % |
| 3. | Poloxamer 407 | 1.98 % |
| 4. | Flavouring | 1.10 % |
| 5. | Chlorhexidine di-gluconate | 1.32 % Theoretical in reconstituted sol: 0.120 % |

### ANALYTICAL RESULTS

Appearance: homogeneous white powder **(APPROVED)**
pH: 6.61 (*) **(APPROVED)**
Density of solid (g/ml): 0,680 **(APPROVED)**
Solubility: (*) **TRANSPARENT** solution in any kind of water **(APPROVED)**
CHX-dg (in reconstituted solution): 0.117 % (Recovery %: 97.5) **(APPROVED)**
CHX-dg (in the solid): 1.179 % (Recovery %: 90.0) **(APPROVED)**

### COMPARATIVE EXAMPLE 04:

Internal reference: **S/EML-11-00-A/01** (Product with **CHX-da ONLY)** Composition:

| | | |
|---|---|---|
| 1. | Xylitol | q.s. 100 %. |
| 2. | Sodium gluconate | 11.0 % |
| 3. | Poloxamer 407 | 1.98 % |
| 4. | Flavouring | 1,10 % |
| 5. | Chlorhexidine-di-acetate | 0.92 % Theoretical in reconstituted sol. 0.084 % |

### ANALYTICAL RESULTS

Appearance: homogeneous white powder **(APPROVED)**
pH: 6.64 (*) **(APPROVED)**
Density of solid (g/ml): 0,710 **(APPROVED)**
Solubility: (*) **TRANSPARENT** solution in any kind of water **(APPROVED)**
CHX-da (in reconstituted solution): 0.075 % (Recovery %: 90.0) **(APPROVED)**
CHX-da (in the solid): 0.831 % (Recovery %: 90.3) **(APPROVED)**

### COMPARATIVE EXAMPLE 05:

Internal reference: **S/EML-12-00-A/01** (Product **WITHOUT CHX and WITHOUT CPC)** It was manufactured as in the case of using CHX-da

### Composition:

| | | |
|---|---|---|
| 1. | Xylitol | q.s. 100 % |
| 2. | Sodium gluconate | 11.0 % |
| 3. | Poloxamer 407 | 1.98 % |
| 4. | Flavouring | 1.10 % |

### ANALYTICAL RESULTS

Appearance: homogeneous white powder **(APPROVED)**
pH: 6.69 (*) **(APPROVED)**
Density of solid (g/ml): 0.720 **(APPROVED)**
Solubility: (*) **TRANSPARENT** solution in any kind of water **(APPROVED)**
CPC (in reconstituted solution): 0.047 % (Recovery %: 94.0) **(APPROVED)**
CPC (in the solid): 0.511 % (Recovery %: 92.9) **(APPROVED)**

### EXAMPLE 06:

Internal reference: **S/EML-08-00-A/01** (Product with **CHX-da** + **CPC)**

### Composition:

| | | |
|---|---|---|
| 1. | Xylitol | q.s. 100 % |
| 2. | Sodium gluconate | 11.0 % |
| 3. | Poloxamer 407 | 1.98 % |
| 4. | Flavouring | 1.10 % |
| 5. | Chlorhexidine diacetate | 0.92 % Theoretical in reconstituted sol: 0.084 % |
| 6. | CPC | 0.55 % Theoretical in reconstituted sol.: 0.050 % |

### ANALYTICAL RESULTS

Appearance: homogeneous white powder **(APPROVED)**
pH: 6.71 (*) **(APPROVED)**
Density of solid (g/ml): 0.720 **(APPROVED)**
Solubility (*): **TRANSPARENT** solution in any kind of water **(APPROVED)**
CHX-da (in reconstituted solution): 0.088 % (Recovery %: 105.4) **(APPROVED)**
CHX-da (in the solid): 0.863 % (Recovery %: 106.6) **(APPROVED)**
CPC (in reconstituted solution): 0.052 % (Recovery %: 104.0) **(APPROVED)**
CPC (in the solid): 0.560 % (Recovery %: 101.8) **(APPROVED)**

### EXAMPLE 07:

Internal reference: **S/EML-13-00-A/01** (Product with **CHX-da** + **CPC** + **L-Leucine)** Composition:

| | | |
|---|---|---|
| 1. | Xylitol | q.s. 100 % |
| 2. | Sodium gluconate | 11.0 % |
| 3. | L-Leucine | 5.00 % (as fluidizer) |
| 4. | Poloxamer 407 | 1.98 % |
| 5. | Flavouring | 1.10 % |
| 6. | Chlorhexidine diacetate | 0.92 % Theoretical in reconstituted sol: 0.084 %. |
| 7. | CPC | 0.55 % Theoretical in reconstituted sol.: 0.050 %. |

### ANALYTICAL RESULTS

Appearance: homogeneous white powder **(APPROVED)**
pH: 6.71(*) **(APPROVED)**
Density of solid (g/ml): 0.730 **(APPROVED)**
Solubility (*): **TRANSPARENT** solution in any kind of water **(APPROVED)**
CHX-da (in reconstituted solution): 0.086 % (Recovery %: 102.4) **(APPROVED)**
CHX-da (in the solid): 1.006 % (Recovery %: 109.3) **(APPROVED)**
CPC (in reconstituted solution): 0.052 % (Recovery %: 104.0) **(APPROVED)**
CPC (in the solid): 0.611 % (Recovery %: 111.1) **(APPROVED)**

### EXAMPLE 08:

Internal reference: **S/EML-15-00-A/01** (Product with **CHX-dg** + **CPC** + **L-Leucine)**

### Composition:

| | | |
|---|---|---|
| 1. | Xylitol | q.s. 100 % |
| 2. | Sodium gluconate | 11.0 % |
| 3. | L-Leucine | 5.00 % (as fluidizer) |
| 4. | Poloxamer 407 | 1.98 % |
| 5. | Flavouring | 1.10 % |
| 6. | Chlorhexidine di-gluconate | 1.32 % Theoretical in reconstituted sol: 0.120 % |
| 7. | CPC | 0.55 % Theoretical in reconstituted sol.: 0.050 %. |

### ANALYTICAL RESULTS

Appearance: homogeneous white powder **(APPROVED)**
pH: 6.61 (*) **(APPROVED)**
Density of solid (g/ml): 0.690 **(APPROVED)**
Solubility (*): **TRANSPARENT** solution in any kind of water **(APPROVED)**
CHX-dg (in reconstituted solution): 0.117 % (Recovery %: 97.5) **(APPROVED)**
CHX-dg (in the solid): 1.402 % (Recovery %: 106.2) **(APPROVED)**
CPC (in reconstituted solution): 0.053 % (Recovery %: 96.4) **(APPROVED)**
CPC (in the solid): 0.628 % (Recovery %: 114.2) **(APPROVED)**

### COMPARATIVE EXAMPLE 09:

Internal reference: **S/EML-09-00-A/01** (Product with **CPC and WITHOUT CHX)** It was manufactured as in the case of using CHX-da Composition:

| | |
|---|---|
| 1. Xylitol | q.s. 100 % |
| 2. Sodium gluconate | 11.0 % |
| 3. Poloxamer 407 | 1.98 % |
| 4. Flavouring | 1.10% |
| 5. CPC | 0.55 % Theoretical in reconstituted sol.: 0.050 %. |

### ANALYTICAL RESULTS

Appearance: homogeneous white powder **(APPROVED)**
pH: 6.63 (*) **(APPROVED)**
Density of solid (g/ml): 0.670 **(APPROVED)**
Solubility: **TRANSPARENT** solution in any kind of water **(APPROVED)**
CPC (in reconstituted solution): 0.051 % (Recovery %: 102) **(APPROVED)**
CPC (in the solid): 0.504 % (Recovery %: 91.6) **(APPROVED)**

### EXAMPLE 10:

Internal reference: **S/EML-17-00-A/01** (Product with **CHX-dg** + **CHX da** + CPC)

### Composition:

| | | |
|---|---|---|
| 1. | Xylitol | q.s. 100 %. |
| 2. | Sodium gluconate | 11.0 %. |
| 3. | Poloxamer 407 | 1.98 %. |
| 4. | Flavouring | 1.10%. |
| 5. | Chlorhexidine di-gluconate | 0.66 % Theoretical in reconstituted sol. 0.060 %. |
| 6. | Chlorhexidine diacetate | 0.46 % Theoretical in reconstituted sol: 0.042 % |
| 7. | CPC | 0.55 % Theoretical in reconstituted sol.: 0.050 % |

### ANALYTICAL RESULTS

Appearance: homogeneous white powder **(APPROVED)**
pH: 6,61 (*) **(APPROVED)**
Density of solid (gr/ml): 0.686 **(APPROVED)**
Solubility: **TRANSPARENT** solution in any kind of water **(APPROVED)**
TOTAL CHX (as CHX di-gluconate) in reconstituted solution: 0.121 % (Recovery %: 100.8) **(APPROVED)**
TOTAL CHX (as CHX di-gluconate) in (in solid): 1.240 % (Recovery %: 93.9) **(APPROVED)**
CPC (in reconstituted solution): 0.049 % (Recovery %: 98.0) **(APPROVED)**
CPC (in solid): 0.536 % (Recovery %: 97.4) **(APPROVED)**

### NOTES:

(*) : parameter measured in a solution obtained by dissolving 1,5 g of solid product in 15,0 g of deionised water of analytical grade or multiples thereof.

### C. Antimicrobial effectiveness analysis of the example compositions

The following table shows the different formulations as evaluated and the results of bacterial counts in log 10 cfu/mL after treatment of multispecies oral biofilms grown under *in vitro* conditions.

**Table 1.**

| **EXAMPLE** | **REFERENCE** | **DESCRIPTION** | **BACTERIAL COUNTS (+/- SD)** |
|---|---|---|---|
| Commercial Liquid Formula | na | Liquid formulation with **CHX Di-Gluconate** + **CPC** | 1.86 +/- 2.73 |
| **2** | S/EML-07-00-A/01 | Solid formulation with **CHX Di-Gluconate** + **CPC** | 2.84 +/- 2.93 |
| **6** | S/EML-08-00-A/01 | Solid formulation with **CHX Di-Acetate** + **CPC** | 2.05 +/- 2.77 |
| **3** | S/EML-10-00-A/01 | Solid formulation BLANK **WITHOUT CPC and WITH CHX Di-Gluconate** | 6.8 +/- 0.87 |
| **4** | S/EML-11-00-A/01 | Solid formulation BLANK **WITHOUT CPC and WITH CHX Di-Acetate** | 6.17 +/- 1.11 |
| **5** | S/EML-12-00-A/01 | Solid formulation FULL BLANK **WITHOUT CPC and WITHOUT CHX** | 7.62 +/- 0.13 |

Figure 1 plots the results presented in Table 1. In addition to show the total counts (log10 cfu/mL) of bacteria grown after treatment of the biofilms with the various solid compositions with CHX and CPC and the standard *gold* liquid formulation, the % survival is also presented.

Statistical analysis revealed that all compositions were significantly more effective than the negative control.

The commercial liquid formulation is significantly more effective than the formulations of Examples 3, 4 and 5. And it is not significantly different from the formulations of Examples 2 and 6.

The composition of Example 2 is significantly more effective than the compositions of Examples 3, 4 and 5. And it is not significantly different from that of Example 6.

The composition of Example 6 is significantly more effective than the compositions of Examples 3, 4 and 5. And there are no significant differences with the formulation of Example 2.

There are no statistically significant differences between the formulations Example 2 and Example 6. There are also no statistically significant differences between these formulations and the commercial liquid formulation. All three formulations are statistically equally effective in their antimicrobial capacity against multispecies oral biofilms grown and treated under *in vitro* conditions.

The composition of Example 3 is significantly more effective than the formulation of Example 5.

There are no significant differences in antimicrobial effectiveness between the compositions of Examples 3 and 4.

### Summary of antiseptic activities of the different compositions

### A. Examples with the combination CPC + CHX dq

**S/EML-07-00-A/01 (Example 2):** significantly more effective than S/EML-10-00-A/01 (Example 3 - blank WITHOUT CPC and with CHX dg)
**S/EML-07-00-A/01 (Example 2):** significantly more effective than S/EML-11-00-A/01 (Example 4 - blank WITHOUT CPC and with CHX da)
**S/EML-07-00-A/01 (Example 2):** significantly more effective than S/EML-12-00-A/01 (Example 5 - full blank WITHOUT CPC and WITHOUT CHX)
**S/EML-07-00-A/01 (Example 2):** no significant difference S/EML-08-00-A/01 (Example 6 - CPC + CHX da)

Activity of S/EML-07-00-A/01 (Example 2 -CPC + CHX dg) = S/EML-08-00-A/01 = Liquid commercial formulation.

### B. Examples with the combination CPC + CHX da

**S/EML-08-00-A/01 (Example 6):** significantly more effective than S/EML-10-00-A/01 (Example 3 - blank WITHOUT CPC and with CHX dg)
**S/EML-08-00-A/01 (Example 6):** significantly more effective than S/EML-11-00-A/01 (Example 4 - blank WITHOUT CPC and with CHX da)
**S/EML-08-00-A/01 (Example 6):** significantly more effective than S/EML-12-00-A/01 (Example 5 - full blank WITHOUT CPC and WITHOUT CHX)
**S/EML-07-00-A/01 (Example 6):** no significant difference S/EML-07-00-A/01 (Example 2 - CPC + CHX da)

Activity of S/EML-08-00-A/01 (Example 6 -CPC + CHX dg) = S/EML-07-00-A/01 (Example 2 - CPC + CHX dg) = Commercial liquid formulation.

## Claims

1. A solid composition comprising chlorhexidine digluconate and/or diacetate and cetylpyridinium chloride for use in the prevention and/or treatment of oral infectious pathologies.

2. The solid composition for use according to claim 1, which is in the form of a powder, granulate or tablet.

3. The solid composition for use according to any one of claims 1 or 2, wherein the chlorhexidine digluconate, when present as the only chlorhexidine salt, is at a concentration of 0.220-2.200 % w/w.

4. The solid composition for use according to any one of claims 1 or 2, wherein the chlorhexidine diacetate, when present as the only chlorhexidine salt, is present at a concentration of 0.154-1.540 % w/w.

5. The solid composition for use according to any one of claims 1 or 2, wherein when a mixture of chlorhexidine diacetate and chlorhexidine digluconate is present, the amount of total chlorhexidine, expressed as chlorhexidine digluconate, is present at a concentration of 0.220 -2.200 % w/w.

6. The solid composition for use according to any one of claims 1 to 5, wherein cetylpyridinium chloride is present at a concentration of 0.133-1.100 % w/w.

7. The solid composition for use according to any one of claims 1 to 6, wherein said composition is dissolved in water for reconstitution into a liquid for application by means of an oral irrigator for extemporaneous use or mouthwash for extemporaneous use.

8. The solid composition for use according to any one of claims 1 to 6, wherein said prevention and/or treatment of oral infectious pathologies is performed by directly administering the solid composition to the oral cavity.

9. The solid composition for use according to any one of claims 1 to 8, wherein said oral infectious pathologies include:
- caries in primary and/or permanent teeth;
- post-operative infections in the oral cavity;
- oral halitosis;
- gingivitis
- periodontitis;
- oral mucositis;
- peri-implant mucositis;
- peri-implantitis
- aphtha;
- fungi;
- sexually transmitted infections in the oral cavity;
- oral infectious pathologies affecting aspiration pneumonias in intubated and/or Intensive Care Unit patients;
- oral infectious pathologies affecting aspiration pneumonias in patients with Gastroesophageal Reflux Disease;
- infected oral ulcers;
- herpetic gingivostomatitis.
